# FASCICULE DE BREVET EUROPEEN

(11) **EP 3 394 622 B1**
(45) Date de publication et mention de la délivrance du brevet: **04.08.2021**
(21) Numéro de dépôt: 16826123.8
(22) Date de dépôt: 20.12.2016
(51) Int. Cl.: G01N 33/68, G01N 33/53, C08F 220/60

(54) **DILUANT DE REACTIFS**
REAGENZVERDÜNNUNGSMITTEL
REAGENT DILUENT

(30) Priorité: 21.12.2015 FR 1562932
(43) Date de publication de la demande: 31.10.2018
(73) Titulaire: BIOMÉRIEUX, 69280 Marcy L'Etoile (FR)
(72) Inventeur: BUSSERET, Sandrine, 69001 Lyon (FR); BETTSWORTH, Florence, 69280 Marcy l'étoile (FR); MARTINEZ, Jérôme, 69009 Lyon (FR)
(74) Mandataire: Plasseraud IP
(86) Numéro de dépôt international: PCT/FR2016/053566
(87) Numéro de publication internationale: WO 2017/109376

(56) Documents cités:
- EP-A1- 1 314 982
- J.-L. POPOT ET AL: "Amphipols From A to Z*", ANNUAL REVIEW OF BIOPHYSICS, vol. 40, no. 1, 9 juin 2011 (2011-06-09), pages 379-408, XP055276256, ISSN: 1936-122X, DOI: 10.1146/annurev-biophys-042910-155219
- MARTIN PICARD ET AL: "Protective and Inhibitory Effects of Various Types of Amphipols on the Ca 2+ -ATPase from Sarcoplasmic Reticulum: A Comparative Study +", BIOCHEMISTRY, vol. 45, no. 6, 1 février 2006 (2006-02-01), pages 1861-1869, XP055276260, US ISSN: 0006-2960, DOI: 10.1021/bi051954a
- KAZUHIKO ISHIHARA ET AL: "WHY DO PHOSPHOLIPID POLYMERS REDUCE PROTEIN ADSORPTION?", JOURNAL OF BIOMEDICAL MATERIALS RESEARCH, WILEY, NEW YORK, NY, US, vol. 39, no. 2, 1 janvier 1998 (1998-01-01), pages 323-330, XP002972153, ISSN: 0021-9304, DOI: 10.1002/(SICI)1097-4636(199802)39:2<323::A ID-JBM21>3.0.CO;2-C

## Description

La présente invention concerne le domaine du diagnostic ou du pronostic. En particulier, elle concerne des compositions utiles lors de la mise en œuvre d'immunoessais, notamment comme agent stabilisant et/ou bloquant.

### ETAT DE LA TECHNIQUE

Les immunoessais sont utilisés couramment dans les domaines d'analyses cliniques, alimentaires, pharmaceutiques et chimiques. Ainsi, ils ont pour but de déterminer la présence d'un ou plusieurs analytes dans des échantillons susceptibles de les contenir. L'immunoessai est un test largement connu de l'Homme du Métier qui implique des interactions spécifiques entre l'analyte à détecter et un ou des partenaire(s) de liaison à cet analyte. A titre d'exemple de tels immunoessais, on peut citer les méthodes telles qu'ELISA (Enzyme Linked Immuno Sorbent Assay), ELFA (Enzyme Linked Florescent Assay) et RIA (Radio Immuno Assay) qui peuvent fonctionner selon le principe du « sandwich », ou encore selon le principe de la « compétition », et les méthodes d'immunodétection comme l'immunohistochimie, l'immunocytochimie, l'immunofluorescence, le Western-blot et le Dot-blot. Les méthodes en « compétition » sont habituellement utilisées pour des petites molécules telles que les haptènes, les méthodes « sandwich » étant utilisées pour les autres analytes.

Les immunoessais comprennent la mise en contact de l'échantillon susceptible de contenir un analyte avec au moins un partenaire de liaison marqué ou un réactif de compétition à l'analyte marqué. Ledit partenaire de liaison ou réactif de compétition est par exemple formé d'un anticorps conjugué à un marqueur susceptible de délivrer un signal. Ces réactifs peuvent être mis en solution et conservés dans un diluant avant la mise en œuvre de l'immunoessai.

Quel que soit le type d'interactions détectées, l'adsorption sur la matrice solide, sur les partenaires de liaison ou sur le réactif de compétition, de réactifs qui ne doivent pas s'y attacher directement, peut contribuer à générer un bruit de fond. Il est donc essentiel de diminuer ces interactions non-spécifiques afin de diminuer autant que possible le bruit de fond.

On utilise pour cela un agent bloquant ou agent saturant qui a pour fonction de saturer les sites après l'adsorption.

Les diluants de réactifs pour immunoessai peuvent ainsi comprendre des agents bloquants et/ou stabilisants, d'une part pour stabiliser les réactifs (conjugués, marqueurs) et d'autre part pour réduire les interactions non-spécifiques du ou des réactifs, par exemple avec la matrice solide, les partenaires de liaison et/ou un réactif de compétition.

Ces agents bloquants sont de préférence choisis parmi les protéines d'origine animale ou humaine, et notamment les sérums animaux, le lait écrémé, notamment en poudre, les caséines hydrolysées ou non, l'albumine bovine, humaine et les gélatines.

Ces produits d'origine biologique, et notamment animale ou humaine, ont l'inconvénient de présenter une composition variable selon les lots. En effet, pour les sérums animaux, leur composition peut varier en fonction de l'espèce, de la race, du sexe, ainsi que de leur alimentation (variation saisonnière). Pour les protéines purifiées comme l'albumine bovine par exemple, selon le procédé de purification utilisé qui est dépendant du fournisseur, la qualité de ces albumines est variable. Des contaminations, comme la présence de protéases, d'immunoglobulines bovines ou autres molécules en quantités plus ou moins importantes, sont à l'origine des variations sur le bruit de fond. Ces matières premières d'origine humaine ou animale conservent leur pouvoir stabilisant mais le pouvoir bloquant est variable d'un lot à l'autre. De plus, leur mise en œuvre doit suivre les normes de sécurité relatives aux risques biologiques et présentent également des contraintes en termes d'approvisionnement, de stockage des produits sources et de leur manipulation. Par conséquent, pour remédier à ces inconvénients, il serait avantageux d'utiliser des agents stabilisants et/ou bloquants synthétiques, utiles dans des compositions pour immunoessais, et notamment dans des diluants de réactifs pour immunoessais, tels que des anticorps marqués.

Des copolymères amphotériques de 2-méthacryloyloxyéthyl phosphorylcholine, sont décrits comme agents stabilisants et/ou bloquants dans des immunoessais de type ELISA (Sakaki et al., 1999 J.Biomed.Mater.Res., 47, 523 ; Sakaki et al., 2000, Polymer Journal, Vol 32, No. 8, pp 637-641). Les copolymères de 2-méthacryloyloxyéthyl phosphorylcholine sont commercialisés par la société NOF Corporation sous le nom commercial Biolipidure™ ou Lipidure®. Ils sont présentés comme une alternative à l'albumine bovine notamment pour diminuer les interactions non-spécifiques et/ou stabiliser les anticorps -marqués (EP1314982 A). Kasuhiko Ishira et al. ont étudié la diminution de l'absorption de protéines en présence de polymères phospholipides (Journal of Biomédical materials research, vol. 39, nO. 2, 1 janvier 1998, pages 323-330.

Les demandes de brevet WO 98/27434 et WO2008/058963 décrivent l'utilisation de polymères amphiphiles comme solvant et agent stabilisant de protéines membranaires ou dans des applications de vectorisation. L'utilisation de ces composés, de la famille des amphipols, comme agent stabilisant d'anticorps thérapeutiques a également été décrite dans la demande WO2010/073119. J.-L. Popot et al. ont publié une revue sur les amphipols (Annual Review of Biophysics, vol. 40, no. 1, 9 juin 2011, pages 379-408). Les effets protecteurs et inhibiteurs de différents types d'amphipols ont été étudiés par Picard et al. (Biochemistry, vol. 45, no. 6, 1 février 2006, pages 1861-1869).

La Demanderesse a maintenant mis en évidence qu'une composition comprenant l'association combinée d'un amphipol et d'un copolymère amphotérique de (méth)acrylate comprenant au moins un groupe phosphorylcholine peut être utilisée avantageusement comme agent stabilisant et/ou bloquant de réactifs, notamment en remplacement de certains agents stabilisants et/ou bloquants d'origine animale classiquement utilisés.

En outre, et de manière tout à fait surprenante, la Demanderesse a mis en évidence que l'utilisation de ces compositions selon l'invention dans un test d'immunoessai permet de diminuer sensiblement le signal de bruit de fond contribuant ainsi à augmenter la sensibilité du test, tout en maintenant la stabilité des réactifs dans le temps.

### RESUME

Ainsi, la présente invention a pour objet une composition pour immunoessai, comprenant au moins (i) un amphipol et (ii) un copolymère amphotérique à base de monomères de (méth)acrylate, une partie de ces monomères comprenant un groupe phosphorylcholine.

En particulier, l'amphipol peut être un polymère amphiphile comprenant un homopolymère ou copolymère d'anhydride maléique sur lesquels sont greffés au moins un groupe hydrophobe et un groupe hydrophile, et d'une masse moléculaire moyenne comprise entre 1 000 et 100 000, de préférence entre 4 000 et 70 000 g.mol⁻¹.

Dans un mode de réalisation spécifique, l'amphipol est choisi parmi les poly(anhydride-alt-1-octadécène maléiques), poly(anhydride-alt-1-tétradécène maléiques), poly(anhydride-alt-1-décène maléiques), étant substitués par un groupe hydrophile, par exemple un ammonium alkylé, et notamment la 3-(diméthylamino)-1-propylamine.

Dans un autre mode de réalisation spécifique, éventuellement combiné avec les précédents modes de réalisation spécifiques, la composition selon l'invention est caractérisée en ce que le copolymère amphotérique est un copolymère amphotérique à base d'un monomère de 2-méthacryloyloxyéthyl phosphorylcholine (MPC) et d'un autre monomère de méthacrylate fonctionnalisé avec un groupe hydrophobe, un groupe anionique, un groupe cationique ou un groupe permettant de former une ou plusieurs liaisons hydrogène.

Dans un mode de réalisation spécifique, le copolymère amphotérique est un copolymère amphotérique à base de MPC et de méthacrylate fonctionnalisé avec un groupe hydrophobe choisi parmi
- les alkyles linéaires ou ramifiés en C₄ à C₃₀ ;
- les alkyles linéaires ou ramifiés en C₄ à C₃₀ comprenant au moins un cycloalkyle ;
- les chaînes hydrocarbonées en C₄ à C₃₀ comprenant au moins une double liaison ;
- les chaînes hydrocarbonées en C₄ à C₃₀ comprenant au moins une triple liaison ; ou,
- les chaînes hydrocarbonées en C₄ à C₃₀ comprenant au moins un cycle aromatique.

Par exemple, il s'agit des Biolipidures™ ou Lipidure®, par exemple le Biolipidure™ 206 commercialisé notamment par la société NOF Corporation.

L'invention vise également une solution aqueuse comprenant les compositions selon l'invention et au moins un réactif d'immunoessai. En particulier, dans un mode de réalisation préféré, la solution aqueuse selon l'invention est caractérisée en ce qu'elle ne comprend pas d'agent stabilisant et/ou d'agent bloquant d'origine humaine ou animale, par exemple choisi parmi les sérums animaux, le lait écrémé, notamment le lait écrémé en poudre, les caséines hydrolysées ou non, l'albumine bovine ou humaine et les gélatines. L'invention vise en outre les trousses d'immunoessai comprenant au moins une solution aqueuse selon l'invention, et au moins un partenaire de liaison à un analyte à détecter ou quantifier par immunoessai et, le cas échéant, les composés nécessaires à la mise en évidence d'une interaction spécifique entre le ou les partenaires de liaison et l'analyte.

Les compositions et solutions aqueuses selon l'invention sont utiles en particulier pour réduire l'interaction non-spécifique entre
i. au moins un partenaire de liaison destiné à détecter ou quantifier un analyte dans un échantillon biologique susceptible de le contenir, par exemple un partenaire de liaison marqué, et
ii. une phase solide.

Ou encore, elles sont utiles pour réduire l'interaction non-spécifique entre
i. un contrôle positif de l'immunoessai, un standard de l'immunoessai et/ou un ajusteur de l'immunoessai, et
ii. une phase solide.

Enfin, l'invention vise un procédé par immunoessai de détermination de la présence ou de quantification d'un analyte cible, dans un échantillon biologique susceptible de le contenir, comprenant la mise en contact dudit échantillon avec au moins deux partenaires de liaison audit analyte, P1 et P2, séquentiellement ou simultanément, l'un des deux partenaires P2 étant marqué pour révéler la présence dudit analyte, s'il est présent, l'autre partenaire P1 étant de préférence immobilisé sur une phase solide, et ledit partenaire P2 marqué étant contenu dans au moins une composition ou solution aqueuse selon l'invention, pour former le cas échéant un complexe Pl/analyte/P2, la révélation de la présence dudit complexe formé permettant de conclure à la présence ou à la quantité d'analyte dans l'échantillon.

Elle concerne également un procédé par immunoessai de détermination de la présence ou de quantification d'un analyte cible, dans un échantillon biologique susceptible de le contenir, comprenant la mise en contact dudit échantillon avec au moins un partenaire P1 de liaison audit analyte et un réactif R1 de compétition à l'analyte, ledit partenaire de liaison P1 étant de préférence immobilisé sur une phase solide, ledit réactif R1 étant marqué pour révéler indirectement la présence dudit analyte, et ledit réactif R1 étant contenu dans au moins une composition ou solution aqueuse selon l'invention, pour former un complexe P1/R1, la révélation de la présence dudit complexe formé permettant de conclure indirectement à l'absence ou à la quantité d'analyte dans l'échantillon.

### DESCRIPTION DETAILLEE

La Demanderesse a donc mis au point contre toute attente des compositions pour immunoessai permettant de remplacer les macromolécules d'origine animale classiquement utilisées dans les diluants de réactifs pour immunoessai.

Les compositions pour immunoessai selon l'invention comprennent (i) un amphipol et (ii) un copolymère amphotérique à base de monomères de (méth)acrylate, une partie de ces monomères comprenant un groupe phosphorylcholine ou plus généralement un groupe de formule (III) décrit plus loin, par exemple une partie de ce monomères étant le monomère de MPC (2- Méthacryloyloxyléthyl-PhosphorylCholine).

### Les amphipols

Au sens de l'invention, le terme « amphipol » fait référence à la famille de polymères amphiphiles courts et flexibles et décrits notamment dans les revues de Zoonens et Popot, 2014 (J. Membrane Biol. 2014, 247(0) : 759-796) et Zoonens et al, 2014 (I. Mus-Veteau (ed.), Membrane proteins Production for Structural Analysis, © Springer Science+ business Media New York 2014, « Chapter 7 Amphipols : A General Introduction and Some Protocols », page 173-203).

Dans un mode de réalisation particulier, les amphipols utilisables dans les compositions selon l'invention sont choisis parmi les polymères amphiphiles comprenant un homopolymère ou copolymère d'anhydride maléique, sur lesquels sont greffés au moins un groupe hydrophobe et un groupe hydrophile, et d'une masse moléculaire moyenne comprise entre 1 000 et 100 000 g/mol, de préférence entre 4 000 et 70 000 g/mol.

Les premiers amphipols décrits dans l'état de la technique sont des polymères de polyacrylate où certaines fonctions carboxylates sont fonctionnalisées avec l'octylamine, ou l'isopropylamine.

La masse moléculaire moyenne de ces poly(anhydride maléiques) est de préférence comprise entre 1 000 et 70 000 g/mol, plus préférentiellement entre 10 000 et 20 000 g/mol.

Dans un autre mode de réalisation spécifique, le groupe hydrophile est un groupe carboxylate ou un groupe chargé positivement, de préférence un ammonium, par exemple un ammonium alkylé de formule suivante : -(CH₂)ₙ-NH(CH₃)₂ avec n étant compris entre 2 et 6, par exemple la 3-(diméthylamino)-1-propylamine.

L'ammonium alkylé est de préférence rattaché à l'anhydride maléique par une fonction amide, ester ou thioester, de préférence une fonction amide.

La chaine alkyle hydrophobe est choisie parmi les chaines alkyles linéaires de formule - (CH₂)ₙ- avec n supérieur ou égal à 4, de préférence supérieur ou égal à 6, de préférence supérieur ou égal à 8, et par exemple compris entre 4 et 16, de préférence entre 8 et 12.

Ces polymères d'anhydrides maléiques peuvent être choisis en particulier parmi les poly(anhydride-alt-1-octadécène maléiques), les poly(anhydride-alt-1-tétradécène maléiques) et les poly(anhydride-alt-1-décène maléiques), substitués par un groupe hydrophile, de préférence un groupe chargé positivement tel que défini plus haut, et notamment un ammonium alkylé, tel que la 3-(diméthylamino)-1-propylamine.

Dans un mode de réalisation spécifique, l'amphipol utilisé selon l'invention est le poly(anhydride-*alt*-1-tétradécène maléique) substitué de formule (IIa) suivante : 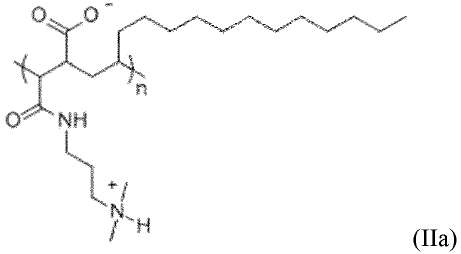 et de masse moléculaire moyenne comprise entre 10 000 et 14 000 g/mol, par exemple environ 12 000 g/mol.

Dans un autre mode de réalisation spécifique, l'amphipol utilisé selon l'invention est le poly(anhydride-*alt*-1-décène maléique) substitué de formule (IIb) suivante : 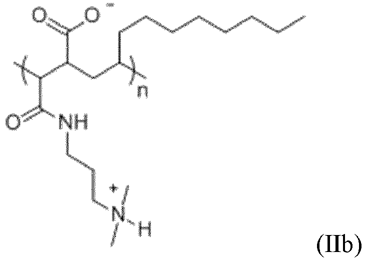 et de masse moléculaire moyenne comprise entre 16 000 et 20 000 g/mol, par exemple environ 18 500 g/mol.

Dans un autre mode de réalisation spécifique, l'amphipol utilisé selon l'invention est le poly(anhydride-*alt*-1-octadécène) substitué de formule (IIc) suivante : 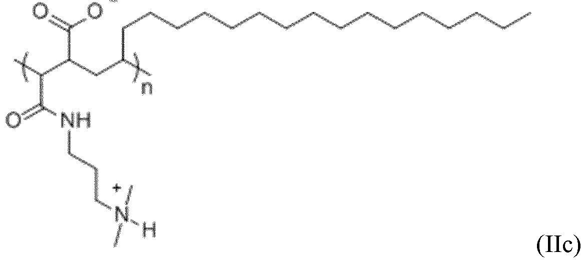 et de masse moléculaire moyenne comprise entre 39 000 et 65 000 g/mol.

Dans un mode préféré, l'amphipol est choisi parmi le PMAL C12 (n° CAS : [869857-14-7]), le PMAL C8 (n° CAS : [869856-84-8]) et le PMAL C16 (n° CAS : [869857-16-9]).

Certains de ces polymères amphipols et notamment de la série PMAL (par exemple PMAL C12, PMAL C8 et PMAL C16) sont fabriqués et commercialisées par la société Anatrace.

### Les polymères amphotériques

Les compositions selon l'invention comprennent un amphipol tel que décrit ci-dessus, en combinaison avec un copolymère amphotérique à base de monomères de (méth)acrylate, une partie comprenant un groupe de formule (III) suivante : 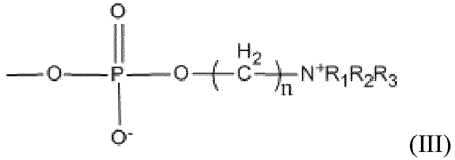 et dans lequel n est un entier de 1 à 6 (de préférence 2),
R₁, R₂, R₃ identiques ou différents, représentent indépendamment l'hydrogène ou un alkyle de 1 à 6 carbones, substitué ou non, de préférence R₁, R₂ et R₃ sont des méthyles.

Par exemple, R₁, R₂, et R₃ selon la formule (IV) peuvent être le méthyle, éthyle, propyle, butyle, isobutyle, pentyle, hexyle. Le nombre de substituants alkyles est de 1 à 3 et les substituants comprennent l'hydroxy ou l'aryle. L'aryle inclut le benzyle ou le naphtyle.

La masse moléculaire moyenne des copolymères amphotériques utilisables selon l'invention est de préférence comprise entre 100 et 1 000 000 g/mol, par exemple entre 1 000 et 500 000 g/mol.

En outre, les copolymères amphotériques incluent un polymère préparé par polymérisation d'un monomère comprenant un groupe de formule (III) avec un autre monomère polymérisable.

Dans un mode de réalisation spécifique, le monomère comprenant un groupe de formule (III) est un monomère comprenant la phosphorylcholine et un groupe vinyle, notamment la 2-acryloyloxyéthyl phosphorylcholine, la 2-méthacryloyloxyéthyl phosphorylcholine (ci-après décrit sous l'abréviation MPC), la 2-(méth)acryloyloxyéthoxyéthyl phosphorylcholine, la 6-méth(acryloyloxyhexyl phosphorylcholine, la 10-(méth)acryloyloxyéthoxynonyl phosphorylcholine, l'allyl phosphorylcholine, la butényl phosphorylcholine, l'hexényl phosphorylcholine, l'octényl phosphorylcholine, la décényl phosphorylcholine. Ces monomères peuvent être préparés selon des méthodes connues décrites notamment dans la demande de brevet japonaise No.6325/79, et No. 154591/83.

L'autre monomère polymérisable comprend le (méth)acrylate tel que le (méth)acrylate de méthyle, le (méth)acrylate d'éthyle, le (méth)acrylate de n-butyle, le (méth)acrylate d'isobutyle, le (méth)acrylate de pentyle, le (méth)acrylate d'hexyle, le (méth)acrylate d'heptyle, le (méth)acrylate d'octyle, le (méth)acrylate de tridécyle, le méthacrylate2-d'hydroxyéthyle; un monomère styrène tel que le styrène, le styrène d'alpha-méthyle, le styrène ayant un groupe phényle substitué avec un (ou plusieurs) groupe(s) méthyle(s) et un styrène ayant un groupe phényle substitué par du chlore; de préférence il s'agit de méthacrylate fonctionnalisé.

Ces polymères et leurs synthèses sont décrits également dans la demande de brevet EP 1 314 982 A1.

En particulier, dans un mode de réalisation spécifique, on utilisera des polymères amphotériques à base de monomères de (méth)acrylate, une partie de ces monomères comprenant un groupe phosphorylcholine.

Le pourcentage de monomères comprenant le groupe phosphorylcholine est de préférence entre 1 et 100%, par exemple entre 1 et 50% ou entre 5 et 10%.

De préférence, il s'agit d'un copolymère amphotérique à base de 2-méthacryloyloxyéthyl phosphorylcholine (MPC), notamment un monomère de méthacrylate comprenant un groupe phosphorylcholine et un autre monomère de méthacrylate comprenant un groupe hydrophobe, un groupe anionique, un groupe cationique et/ou un groupe permettant la formation de liaisons hydrogène.

Un groupe hydrophobe est choisi parmi
- les alkyles linéaires ou ramifiés en C₄ à C₃₀ ;
- les alkyles linéaires ou ramifiés en C₄ à C₃₀ comprenant au moins un cycloalkyle ;
- les chaînes hydrocarbonées en C₄ à C₃₀ comprenant au moins une double liaison ;
- les chaînes hydrocarbonées en C₄ à C₃₀ comprenant au moins une triple liaison ; ou,
- les chaînes hydrocarbonées en C₄ à C₃₀ comprenant au moins un cycle aromatique.

De préférence, le groupe hydrophobe est choisi parmi les chaines alkyles linéaires de formule -(CH₂)ₙ- avec n supérieur ou égal à 4, de préférence supérieur ou égal à 6, de préférence supérieur ou égal à 8, et par exemple compris entre 4 et 16, de préférence entre 8 et 12.

Un groupe anionique peut être choisi en particulier parmi ceux comprenant un radical carboxylate et en particulier les alkylcarboxylates en (C₁-C₅), ceux comprenant un radical sulfonate, et en particulier les alkylsulfonates en (C₁-C₅) et ceux comprenant un radical phosphonate, et en particulier les alkylphosphonates en (C₁-C₅).

Un groupe cationique peut être choisi en particulier parmi un radical (CH₂)ₙ-NR₉R₁₀R₁₁, n étant un nombre entier de 1 à 5 et R₉, R₁₀ et R₁₁, identiques ou différents étant l'hydrogène ou une chaine alkyle (C₁-C₄).

Les composés permettent de former des liaisons hydrogène sont largement connue de l'Homme du Métier. Un exemple de groupe permettant la formation de liaisons hydrogène comprend les aminés, les alcools, les thiols, les aldéhydes, les acides ou les cétones.

Des copolymères de MPC préférés sont commercialisés en particulier par la société NOF Corporation sous la dénomination commerciale de Lipidure® ou Biolipidure® et, notamment, de formule générale (IV) suivante : 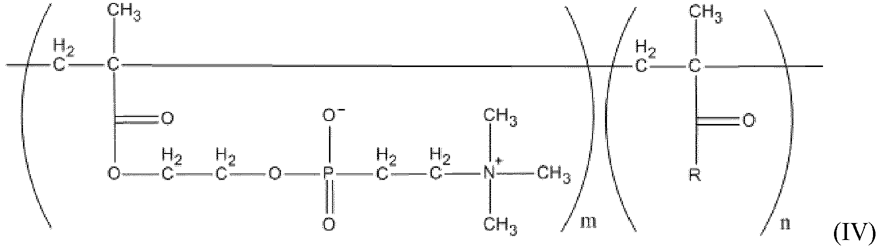 dans laquelle R est un groupe hydrophobe comme défini plus haut, avec m et n correspondant aux pourcentages respectifs des deux monomères et m est compris entre 1 et 99%, par exemple entre 1 et 50% ou entre 1 et 10%, et n est compris entre 1 et 99%, par exemple entre 50 et 99% ou entre 90 et 99%.

Selon un mode réalisation plus spécifique, le polymère amphotérique de 2-méthacryloyloxyéthyl phosphorylcholine (MPC) est un copolymère amphotérique de MPC et de méthacrylate fonctionnalisé avec une chaine alkyle linéaire de formule (-CH₂)ₙ- avec n supérieur ou égal à 4, ou supérieur ou égal à 6, ou supérieur ou égal à 8, et par exemple compris entre 4 et 16, par exemple entre 8 et 12.

A titre d'exemple préféré, pour la réalisation des compositions pour immunoessai selon l'invention, on choisira le Biolipidure™ 206, le Biolipidure™ 203, le Biolipidure™ 802 ou encore le Biolipidure™ 1201, commercialisés par la société NOF Corporation.

### Utilisation des compositions selon l'invention

Les compositions pour immunoessai selon l'invention décrites ci-dessus sont avantageusement utilisées comme agent stabilisant et/ou bloquant.

Au sens de l'invention, par « agent stabilisant », on entend un composé ou composition capable de favoriser la stabilité (thermique et dans le temps) du partenaire de liaison (qui peut être dénaturé après conservation à température ambiante par exemple), de réduire sa dégradation par hydrolyse, de prévenir son agrégation et/ou plus généralement de contribuer au maintien de la conformation native du partenaire de liaison et de sa capacité à lier l'analyte à détecter. La stabilité thermique d'un réactif d'immunoessai peut être déterminée en comparant le signal de détection avant son stockage à température ambiante et après conservation à température ambiante pendant 30 jours. Un réactif est stable si le signal mesuré à 30 jours est dans des valeurs proches du signal mesuré à t0, par exemple comprises entre 90% et 110% du signal mesuré à t0.

Par « agent bloquant », on entend un composé ou composition susceptible de réduire les interactions non-spécifiques entre un partenaire de liaison et une molécule non-spécifique d'un immunoessai, et donc de réduire le signal obtenu en présence d'un contrôle négatif (« bruit de fond »).

En particulier, les compositions selon l'invention sont utiles pour réduire l'interaction non-spécifique entre au moins un réactif d'immunoessai, de préférence un partenaire de liaison, marqué ou pas, et une phase solide.

Dans un autre mode de réalisation, les compositions aqueuses selon l'invention sont utiles pour réduire l'interaction non-spécifique entre
(i) un contrôle positif de l'immunoessai et/ou un standard de l'immunoessai et/ou un ajusteur de l'immunoessai, et
(ii) une phase solide.

Le terme « interaction non-spécifique » s'entend par opposition à l'interaction spécifique du partenaire de liaison avec l'analyte.

Par interaction spécifique, on entend une préférence de liaison (affinité) d'un partenaire de liaison envers l'analyte cible au moins 2 fois, de préférence au moins 5 fois, et plus préférentiellement au moins 10 ou 20 fois supérieure à celle mesurée envers une molécule non-spécifique (par exemple une ou plusieurs molécules arbitraires qui ne contiennent pas a priori les sites spécifiques de reconnaissance au partenaire de liaison).

Le terme « analyte » désigne ici une substance contenue dans un échantillon, qui doit être détectée, identifiée et/ou quantifiée par une analyse. Il doit donc être compris au sens large comme désignant une substance chimique, biologique ou biochimique. A titre d'exemple d'analytes, on peut citer une protéine, un peptide, un haptène, un polysaccharide ou un oligosaccharide.

Le terme « phase solide » au sens de l'invention fait référence à tout matériel insoluble. La phase solide dans un immunoessai est de préférence choisie pour attirer et immobiliser un réactif de capture. Dans une autre alternative, la phase solide comprend une substance chargée, de charge opposée au réactif de capture, ou à une substance conjuguée au réactif de capture. La phase solide peut être par exemple du plastique, un métal, magnétique ou non-magnétique, du verre ou du silicone, et notamment, un tube à essai, une microplaque, des billes, des microparticules, une puce, un cône ou tout autre configuration connue de l'homme du métier pour la mise en œuvre d'immunoessai.

### Solutions aqueuses comprenant les compositions selon l'invention et un réactif d'immunoessai

Les solutions aqueuses sont obtenues en diluant un réactif d'immunoessai dans un solvant comprenant de l'eau et les compositions selon l'invention contenant les polymères amphipols et amphotériques, telles que décrites aux sections précédentes.

Ainsi, un autre aspect de l'invention vise une solution aqueuse comprenant la composition selon l'invention telle que définie plus haut et au moins un réactif d'immunoessai.

Au sens de l'invention, le terme « réactif d'immunoessai » vise tout réactif destiné à interagir spécifiquement avec un analyte ou un partenaire de liaison.

Bien entendu, le préfixe « immuno » dans le terme « immunoessai », par exemple, n'est pas à considérer dans la présente demande comme indiquant strictement que ledit partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner également des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition étant que le partenaire de liaison concerné soit capable de se lier à l'analyte recherché, de manière spécifique. Ainsi, il est connu de parler de test ELISA pour des tests qui utilisent des partenaires de liaison non immunologiques *stricto sensu,* appelés plus largement en anglais « ligand binding assay », que l'on pourrait traduire en langue française par « test utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

A titre d'exemple de réactif de type partenaire de liaison présent dans les solutions aqueuses selon l'invention, on peut citer les anticorps, les fragments d'anticorps, les nanofitines, les récepteurs, les aptamères, les DARPins ou toute autre macromolécule choisie spécifiquement de manière à obtenir une interaction spécifique avec l'analyte, et de préférence une interaction à haute affinité avec l'analyte, d'un K_{D} tel que mesuré par résonance plasmonique de surface, inférieure à 1µM, notamment inférieur à 100nM et de préférence inférieur à 10nM.

Les anticorps polyclonaux peuvent être obtenus par immunisation d'un animal avec un immunogène, suivie de la récupération des anticorps recherchés sous forme purifiée, par prélèvement du sérum dudit animal, et séparation desdits anticorps des autres constituants du sérum, par exemple par chromatographie d'affinité sur une colonne sur laquelle est fixé un antigène spécifiquement reconnu par les anticorps, notamment l'immunogène, ou à l'aide d'une protéine A ou G.

Les anticorps monoclonaux peuvent être obtenus par la technique des hybridomes largement connue de l'homme du métier. Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')2 ainsi que les scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les aptamères sont des oligonucléotides, généralement ARN ou ADN, identifiés dans des banques contenant jusqu'à 10¹⁵ séquences différentes, par une méthode combinatoire de sélection in vitro appelée SELEX pour « Systematic Evolution of Ligands by Exponentiel Enrichment » (Ellington AD et Szostak JW., 1990, Nature, 346 : 818-822). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celle des anticorps.

Les « DARPins » pour Designed Ankyrin Repeat ProteINS (Boersma YL et Plütckthun A, 2011, Curr. Opin. Biotechnol, 22 : 849-857) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par screening de banques combinatoires.

Dans un mode de réalisation particulier, le réactif d'immunoessai compris dans les solutions aqueuses selon l'invention est choisi parmi un partenaire de liaison destiné à détecter ou quantifier un analyte dans un échantillon biologique susceptible de le contenir, ledit partenaire de liaison pouvant être marqué (par exemple un anticorps marqué) ou non, un contrôle positif, un standard ou un ajusteur d'un immunoessai.

Par « contrôle positif », on entend un composé susceptible de produire un signal conforme au signal attendu par le test d'immunoessai en présence d'un échantillon contenant l'analyte à détecter.

Par « standard », on entend un composé utilisé pour produire une gamme étalon, étape nécessaire pour pouvoir effectuer une quantification d'un analyte. La relation entre les concentrations ou les quantités connues du standard et le signal obtenu par immunoessai est exprimée mathématiquement : c'est la courbe de calibration.

Par « ajusteur », on entend un composé utilisé pour ajuster la mesure de l'immunoessai de l'analyte. Dans ce cas, le signal généré par l'ajusteur et sa concentration est connue. L'ajusteur sert à ajuster une courbe de calibration générée au préalable en tenant compte du vieillissement des réactifs de l'immunoessai ou encore de ces conditions de mise en œuvre.

Le partenaire de liaison présent dans les solutions aqueuses selon l'invention peut être marqué, c'est-à-dire lié, de préférence de manière covalente, directement ou indirectement à un marqueur. Très souvent, le marquage se fait par couplage chimique en utilisant un bras de conjugaison (« cross-linker »). On parle alors de partenaire de liaison conjugué. Un exemple de partenaire de liaison marqué fréquemment utilisé est un anticorps conjugué à une enzyme, appelé aussi anticorps conjugué.

Par marqueur, on entend, notamment, toute molécule capable de générer directement ou indirectement un signal détectable. Une liste non limitative de ces marqueurs de détection consiste en :
- les enzymes qui produisent un signal détectable par exemple par colorimétrie, fluorescence, luminescence, comme la peroxydase de raifort, la phosphatase alcaline, la bêta-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Ainsi, dans un mode particulier de réalisation, la solution aqueuse selon l'invention comprend, outre les polymères décrits aux sections précédentes, un anticorps conjugué à un marqueur, encore appelé « anticorps de détection ».

Des systèmes indirects de détection peuvent aussi être utilisés, comme par exemple des ligands capables de réagir avec un anti-ligand. Le ligand correspond alors au marqueur pour constituer, avec le partenaire de liaison, le conjugué.

Les couples ligand/anti-ligand sont bien connus de l'Homme du Métier, ce qui est le cas par exemple des couples suivants biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits ou être lui-même détectable par un autre couple ligand/anti-ligand, et ainsi de suite.

La quantité de réactifs d'immunoessai présente dans la solution aqueuse selon l'invention dépend de son utilisation finale. Dans un mode de réalisation particulier, le réactif d'immunoessai est un anticorps marqué présent à raison de 100 pg/mL à 20 µg/mL, par exemple de 20 ng/mL à 3 µg/mL.

Les compositions de polymères selon l'invention sont avantageusement utilisées en remplacement des molécules habituellement ajoutées avec les réactifs comme agent stabilisant et/ou agent bloquant, notamment les macromolécules d'origine animale.

Par conséquent, dans un mode de réalisation particulier, la solution aqueuse selon l'invention est caractérisée en ce qu'elle ne comprend pas d'agent stabilisant et/ou d'agent bloquant d'origine animale choisi parmi les sérums animaux, les caséines hydrolysées ou non, le lait écrémé, notamment le lait écrémé en poudre, l'albumine bovine ou humaine et les gélatines, encore appelés de façon générique « protéine de charge » même si des macromolécules autres que des protéines sont utilisées.

La solution aqueuse comprend de préférence l'amphipol et le copolymère amphotérique comme décrits aux précédents paragraphes dans des concentrations efficaces pour agir comme agent bloquant dans un test d'immunessai. Ces concentrations efficaces seront déterminées par l'homme du métier, en particulier en fonction de la concentration de réactifs utilisés, de la nature des réactifs, de l'effet stabilisant et/ou bloquant recherché et/ou du type d'immunoessai.

Typiquement, on utilisera des concentrations finales d'amphipols dans la solution aqueuse comprises entre 0,01% et 0,2% (poids/volume), de préférence entre 0,02% et 0,05%, par exemple 0,02%, et des concentrations finales de polymères amphotériques dans la solution aqueuse comprises entre 0,01% et 0,3% (poids/volume), de préférence entre 0,03% et 0,08%, par exemple 0,05%.

La solution aqueuse peut en outre contenir d'autres constituants et en particulier, un tampon, un détergent, des sels, un colorant, un émollient, un conservateur, etc.

### Trousse d'immunoessai selon l'invention

Les solutions aqueuses de l'invention sont particulièrement utiles pour un procédé de détection d'un analyte, dans un échantillon biologique susceptible de le contenir, comprenant un test d'immunoessai par mise en contact dudit échantillon avec au moins un partenaire de liaison à l'analyte.

Aussi, les trousses d'immunoessai comprenant les solutions aqueuses selon l'invention constituent un autre objet de l'invention.

Les trousses d'immunoessai contiennent un ou plusieurs composés nécessaires à la mise en œuvre d'un test d'immunoessai et en particulier un ou des partenaires de liaison à l'analyte, marqué ou pas, et tous les composés nécessaires à la mise en évidence d'une interaction spécifique entre le ou les partenaires de liaison à l'analyte.

Lorsqu'il s'agit d'un test d'immunoessai de type sandwich, la trousse d'immunoessai comprend alors deux partenaires de liaison, l'un des deux partenaires de liaison étant marqué, pour former le « traceur » ou « partenaire de détection », et l'autre partenaire étant capturé sur une phase solide, encore appelé « partenaire de capture ».

Ces trousses d'immunoessai selon l'invention peuvent comprendre en outre un ou plusieurs des éléments suivants :
- une phase solide, par exemple une microplaque ou des cônes, sensibilisés avec des partenaires de capture, par exemple un anticorps de capture,
- une solution de lavage,
- un partenaire de liaison marqué, par exemple un anticorps conjugué,
- une composition selon l'invention sans réactif d'immunoessai,
- une composition selon l'invention ou solution aqueuse selon l'invention comprenant un réactif à titre de contrôle positif,
- une composition selon l'invention ou solution aqueuse selon l'invention comprenant un ou plusieurs réactifs de concentration connue à titre de standard,
- une composition selon l'invention ou solution aqueuse selon l'invention comprenant un réactif de concentration connue à titre d'ajusteur,
- une notice d'instructions pour la mise en œuvre de l'immunoessai.

Dans un mode particulier de réalisation pour un immunoessai mis en œuvre par un automate, par exemple de type Vidas®, la trousse d'immunoessai selon l'invention est un coffret comprenant les éléments séparés suivants :
- des cônes sensibilisés par des partenaires de liaison, par exemple des anticorps spécifiques de l'analyte à détecter,
- une cartouche, c'est-à-dire une phase solide comprenant une pluralité de puits, par exemple de 4 à 20, dont certains sont de préférence scellés, au moins un des puits comprenant une solution aqueuse selon l'invention, par exemple, une solution aqueuse contenant un réactif, de préférence un anticorps marqué spécifique de l'analyte à détecter,
- le cas échéant, une composition selon l'invention sans réactif d'immunoessai,
- le cas échéant, une composition selon l'invention ou solution aqueuse selon l'invention comprenant un réactif à titre de contrôle positif,
- le cas échéant, une composition selon l'invention ou solution aqueuse selon l'invention comprenant un réactif, de concentration connue, à titre d'ajusteur.

La phase solide et la cartouche en tant que telle, telles que décrites dans les trousses ci-dessus, font également partie de l'invention.

### Procédé de mise en œuvre d'un immunoessai selon l'invention

L'invention vise également tout procédé par immunoessai de détermination de la présence ou de quantification d'un analyte, dans un échantillon biologique susceptible de le contenir, comprenant l'utilisation d'une composition ou solution aqueuse de l'invention notamment comme agent bloquant et/ou stabilisant.

Aussi, un autre objet de l'invention concerne un procédé de détection d'un analyte dans un échantillon biologique susceptible de le contenir, comprenant
i. un test d'immunoessai par mise en contact dudit échantillon avec au moins un partenaire de liaison à l'analyte,
ii. le cas échéant, un test de contrôle de la validité du test d'immunoessai par mise en contact d'un contrôle positif avec lesdits un ou plusieurs partenaires de liaison à l'analyte,
iii. la lecture du test d'immunoessai,
iv. la détermination de la présence dudit analyte dans l'échantillon de test lorsque le signal obtenu par le test d'immunoessai de l'étape i est supérieur à une valeur seuil du test d'immunoessai,
ledit procédé comprenant l'utilisation d'une composition ou solution aqueuse selon l'invention.

Dans un mode de réalisation du procédé de détection ci-dessus, le test d'immunoessai mis en œuvre à l'étape (i) utilise une composition ou solution aqueuse selon l'invention.

Dans un autre mode de réalisation du procédé de détection ci-dessus, le test de contrôle mis en œuvre à l'étape (ii) est obtenu en utilisant une solution aqueuse selon l'invention dans laquelle le réactif est un contrôle.

Naturellement, les deux modes de réalisation précédents peuvent être combinés.

L'échantillon de test dans le cadre de l'invention peut être de diverses origines, par exemple d'origine alimentaire, environnementale, biologique, vétérinaire, clinique, pharmaceutique ou cosmétique.

Parmi les échantillons d'origine alimentaire, on peut citer, de façon non-exhaustive, un échantillon de produits lactés (yaourts, fromages, etc.), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales. Tous ces échantillons, s'ils ne sont pas liquides, sont préalablement traités pour être sous forme liquide.

Tel qu'indiqué précédemment, l'échantillon peut être d'origine environnementale et peut consister, par exemple, en un prélèvement de surface, d'eau, etc.

L'échantillon peut également consister en un échantillon biologique, d'origine humaine ou animale, pouvant correspondre à des prélèvements de fluide biologique (urine, sang total ou dérivés tels que sérum ou plasma, salive, pus, liquide céphalo-rachidien, etc.), de selles (par exemple diarrhées cholériques), de prélèvements de nez, de gorge, de peau, de plaies, d'organes, de tissus ou de cellules isolées, d'échantillons d'écouvillonnage. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon analysé est, en général, susceptible de - ou suspecté de - contenir au moins un analyte représentatif de la présence de microorganismes, d'une maladie, ou d'un état physiologique, à détecter, à caractériser ou à suivre.

Les étapes de ce procédé de détection d'un analyte par immunoessai sont des étapes largement connues de l'Homme du Métier qui ont été décrites précédemment. Notamment, la première étape consiste en la mise en contact de l'échantillon de test avec un ou plusieurs partenaires de liaison à l'analyte, de préférence au moins deux partenaires de liaison pour un test sandwich. Comme décrit précédemment, l'un des deux partenaires peut être couplé à un marqueur pour former un conjugué ou un traceur. L'autre partenaire de liaison peut être capturé sur un support solide comme connu de l'Homme du Métier.

Le signal mesuré émis par le conjugué est alors proportionnel à la quantité d'analyte de l'échantillon biologique.

Le test d'immunoessai i) et le test de contrôle ii) peuvent être mis en œuvre dans n'importe quel ordre, simultanément ou successivement, sur le même support solide ou non.

La lecture du test d'immunoessai est également une étape largement connue de l'Homme du Métier qui dépend du test utilisé.

Enfin, la dernière étape consiste en la détermination de la présence dudit analyte dans l'échantillon de test lorsque le signal obtenu par le test d'immunoessai de l'étape i) est supérieur au seuil de détection du test d'immunoessai. Cette étape est également largement connue de l'Homme du Métier.

Un autre objet de l'invention porte sur un procédé de quantification d'un analyte cible par immunoessai dans un échantillon biologique susceptible de contenir ledit analyte, comprenant
i. un test d'immunoessai par mise en contact dudit échantillon avec au moins un partenaire de liaison à l'analyte,
ii. le cas échéant, un test de contrôle de la validité de l'immunoessai par mise en contact d'un contrôle positif avec lesdits un ou plusieurs partenaires de liaison à l'analyte,
iii. la lecture du test d'immunoessai si le test de contrôle de validité est positif, et
iv. la détermination de la quantité dudit analyte dans l'échantillon de test par comparaison du signal du test d'immunoessai avec une courbe étalon, dans laquelle le réactif est un ajusteur de l'immunoessai,
ledit procédé comprenant l'utilisation d'une composition ou solution aqueuse selon l'invention.

Dans un mode de réalisation du procédé de détection ci-dessus, le test d'immunoessai mis en œuvre à l'étape (i) utilise une composition ou solution aqueuse selon l'invention.

Dans un autre mode de réalisation du procédé de quantification ci-dessus, le test de contrôle mis en œuvre à l'étape (ii) est obtenu en utilisant une solution aqueuse selon l'invention dans laquelle le réactif est un contrôle.

Dans encore un autre mode de réalisation du procédé de quantification ci-dessus, la courbe étalon utilisée à l'étape (iv) est obtenue en utilisant une solution aqueuse selon l'invention dans laquelle le réactif est un ajusteur de l'immunoessai.

Naturellement, les trois modes de réalisation précédents peuvent être combinés.

Bien entendu, la description des échantillons et des diverses étapes de procédé décrites dans le cadre du procédé de détection d'un analyte cible s'applique au procédé de quantification d'un analyte cible. La seule différence consiste en la dernière étape qui consiste à déterminer la quantité d'analyte par comparaison à une courbe étalon, comme expliqué précédemment.

Dans un mode de réalisation spécifique des procédés décrits ci-dessus, le procédé par immunoessai selon l'invention comprend la mise en contact dudit échantillon avec au moins deux partenaires de liaison audit analyte, P1 et P2, séquentiellement ou simultanément, l'un des deux partenaires P2 étant marqué pour révéler la présence dudit analyte, s'il est présent, l'autre partenaire P1 étant de préférence immobilisé sur une phase solide, et ledit partenaire P2 marqué étant contenu dans au moins une composition selon l'invention ou au moins une solution aqueuse selon l'invention, comme décrit précédemment, pour former le cas échéant un complexe P1/analyte/P2, la révélation de la présence dudit complexe formé permettant de conclure à la présence ou à la quantité d'analyte dans l'échantillon.

Dans un autre mode de réalisation, le procédé par immunoessai comprend la mise en contact dudit échantillon avec au moins un partenaire P1 de liaison audit analyte et un réactif R1 de compétition à l'analyte, ledit partenaire de liaison P1 étant de préférence immobilisé sur une phase solide, ledit réactif R1 étant marqué pour révéler indirectement la présence dudit analyte, et ledit réactif R1 étant contenu dans au moins une composition selon l'invention ou au moins une solution aqueuse selon l'invention, pour former un complexe P1/R1, la révélation de la présence dudit complexe formé permettant de conclure indirectement à l'absence ou à la quantité d'analyte dans l'échantillon.

Les mêmes caractéristiques et préférences décrites dans les sections précédentes, notamment quant au choix des polymères et des composés particuliers aux différentes étapes de l'immunoessai s'appliquent également aux procédés de détection et quantification de l'invention.

Les procédés d'immunoessai de l'invention comprennent en particulier la mise en œuvre des trousses d'immunoessai de l'invention décrites précédemment.

L'invention sera mieux comprise à l'aide des exemples suivants qui sont donnés à titre illustratif et non limitatif.

### EXEMPLES

### Comparaison de différentes formulations de diluant de réactifs selon l'invention, pour le dosage de la Troponine I cardiaque

L'étude de diluant d'anticorps conjugués pour un immunodosage de la Troponine I cardiaque a été réalisée avec l'automate d'immunoanalyse VIDAS® (bioMérieux). Le cône à usage unique sert à la fois de phase solide pour la réaction et de système de pipetage. La cartouche de l'automate est composée de 10 puits (X0 à X9) recouverts d'une feuille d'aluminium scellée et étiquetée. Le premier puits (X0) comporte une partie prédécoupée pour faciliter l'introduction de l'échantillon. Le dernier puits (X9) est une cuvette optique dans laquelle la fluorescence du substrat est mesurée. Les différents réactifs nécessaires à l'analyse sont contenus dans les puits intermédiaires. Toutes les étapes du test sont ainsi réalisées automatiquement par l'instrument. Elles sont constituées d'une succession de cycles d'aspiration/refoulement du milieu réactionnel. L'immunodosage de la Troponine I cardiaque a été réalisé par un test sandwich en une étape.

### a) Sensibilisation et passivation des cônes

Les caractéristiques et les fournisseurs des anticorps utilisés sont présentés dans le Tableau 1 ci-dessous. Les cônes ont été sensibilisés avec 300 µL d'une solution des anticorps monoclonaux 19C7 et B90 dilués chacun à 2,5 µg/mL dans un tampon PBS pH 6,2. Après environ 20h d'incubation à +18/25°C avec la solution de sensibilisation, les cônes ont été vidés. Ensuite, 300 µL de cette même solution contenant 10 g/L d'albumine bovine sont ajoutés. La passivation se poursuit à +18/25°C sur la nuit. Les cônes sont vidés, séchés, puis conservés à +4°C jusqu'à utilisation, à l'abri de l'humidité.

**Tableau 1. Anticorps utilisés pour l'immunodosage de la Troponine I cardiaque.**

| **Nom de l'anticorps** | **Cible** | **Fournisseur (Cat. No.)** |
|---|---|---|
| **19C7** | TnI | Hytest |
| | | (4T21-19C7) |
| **B90** | TnI | SDIX |
| | | (B9085MA06-MA) |
| **3D5F7** | TnI | bioMérieux |
| | | (non commercial) |
| **7B9** | TnC | Hytest |
| | | (4T27-7B9) |

L'abréviation TnI correspond à la troponine I cardiaque et l'abréviation TnC correspond à la troponine C cardiaque. L'abréviation Cat. No. correspond à la référence catalogue du fournisseur.

### b) Préparation de diluants pour anticorps conjugués et solutions de conjugués

Différentes solutions de diluant pour anticorps conjugués sont préparées selon le Tableau 2.

**Tableau 2. Composition des différents diluants anticorps conjugués.**

| **Composé** | **Diluant REF** | **Diluant 1** | **Diluant 2** | **Diluant 3** Diluant de l'invention |
|---|---|---|---|---|
| PBS pH 6.4 | **X** | **X** | **X** | **X** |
| Sérum de cheval 5% | **X** | **X** | | |
| Biolipidure 206¹ 0,05% | | | **X** | **X** |
| PMAL C12² 0,02% | | **X** | | **X** |

| | | | | |
|---|---|---|---|---|
| X indique que le composé est présent dans le diluant. ¹ Commercialisé par la société NOF Corporation ref Biolipidure-206. ² CAS No 869857-14-7 commercialisé par la société Anatrace ref P5012. | | | | |

Les diluants REF et 1 à 3 ont été utilisés pour préparer différentes solutions de conjugués. Chaque solution de conjugué contient les 2 anticorps monoclonaux de détection, le 3D5F7 et le 7B9 (décrits au Tableau 1), sous forme de fragments Fab' couplés à la phosphatase alcaline. Ces anticorps conjugués ont été dilués à environ 0,5 µg/mL chacun dans les différents diluants décrits dans le Tableau 2 ci-dessus.

### c) Mode opératoire de l'immunodosage

Les solutions de conjugués ont été testées avec des échantillons naturels (points A, B, C, E et G dont les concentrations en Troponine I sont connues) ou un tampon PBS-BSA comme échantillon blanc (point O).

Dès que le cône VIDAS® est en contact avec l'échantillon, la réaction immunologique commence car les anticorps de capture sont immobilisés sur ce cône. L'automate mélange l'échantillon à tester (135 µL) avec 270 µL de la solution de conjugué.

L'incubation dure environ 9 minutes à 37°C et permet la liaison spécifique de la Troponine I cardiaque aux anticorps adsorbés sur le cône d'une part et aux anticorps conjugués (anticorps de détection) d'autre part. Ensuite, les composants non liés sont éliminés par 3 lavages avec un tampon Tris 200 mM pH 7,8, NaCl 300 mM, Triton X-100 0,2%. Lors de l'étape finale de révélation, le substrat 4-méthylombelliferyl phosphate est aspiré puis refoulé dans le cône ; l'enzyme des anticorps conjugués catalyse la réaction d'hydrolyse de ce substrat en 4-méthylombelliferone dont la fluorescence émise est mesurée à 450 nm. La valeur du signal de fluorescence (RFV=relative fluorescence value) est proportionnelle à la concentration de l'antigène présent dans l'échantillon.

### d) Résultats

Le Tableau 3 récapitule les signaux de fluorescence (RFV=relative fluorescence value) déterminés par l'automate VIDAS® lorsque l'on compare les signaux obtenus avec les anticorps conjugués dilués dans le diluant REF (l'art antérieur), et les différentes formulations de diluants (Tableau 2).

**Tableau 3. Résultats obtenus avec les différentes formulations de diluant.**

| **Item** | **Diluant REF** | **Diluant 1** | **Diluant 2** | **Diluant 3** Diluant de l'invention |
|---|---|---|---|---|
| Signal RFV point O (blanc) | 7,5 | 9,8 | 7,0 | 4,5 |
| Signal RFV point A (4.0 ng/L) | 13 | 13 | 18 | 18 |
| RFV A /RFVO | 1,7 | 1,3 | 2,6 | 4,0 |
| RFV A - RFV O | 5,5 | 3,3 | 11,0 | 13,5 |
| Signal RFV point B (10.2 ng/L) | 26,0 | 25,0 | 31,5 | 33,5 |
| RFV B / RFV O | 3,5 | 2,6 | 4,5 | 7,4 |
| RFV B - RFV O | 18,5 | 15,3 | 24,5 | 29,0 |

Comme observé sur le Tableau 3, l'ajout de PMAL C12 au diluant REF (Diluant 1) n'a quasiment pas d'impact sur les signaux observés. En revanche, le remplacement du sérum de cheval par le Biolipidure 206 (Diluant 2) améliore la dynamique en bas de gamme (signaux des points A et B plus élevés), mais ne permet pas de réduire le bruit de fond (signaux des points O autour de 7 RFV). C'est l'ajout de PMAL C12 à ce diluant (Diluant 3, diluant de l'invention) qui permet de diminuer significativement le bruit de fond qui passe de 7 à 4,5 RFV. Il en résulte une augmentation significative des rapports RFV A / RFV O et RFV B / RFV O. Ainsi, l'association du Biolipidure 206 et du PMAL C12 a un effet synergique inattendu.

### d) Stabilité des anticorps conjugués dilués dans les différentes formulations de diluant

Les anticorps conjugués (anticorps de détection) sont dilués à 0,5 µg/mL dans les différentes formulations de diluant présentées en Tableau 2 et sont utilisées tout de suite pour doser les échantillons (A, B, C, E et G). Les résultats obtenus correspondent aux résultats du temps T0. Les anticorps de détection dilués dans les différents diluants sont ensuite conservés pendant 1 mois, soit à +2/8°C (condition normale), soit à +18/25°C (condition de stabilité accélérée). Au bout d'un mois, les mêmes échantillons sont redosés sur VIDAS dans les mêmes conditions avec les solutions diluées d'anticorps de détection stockées pendant 1 mois. Pour chaque condition, le rapport entre le signal RFV obtenu au bout d'un mois sur le signal RFV obtenu à T0 est calculé. Les résultats sont présentés en Tableau 4.

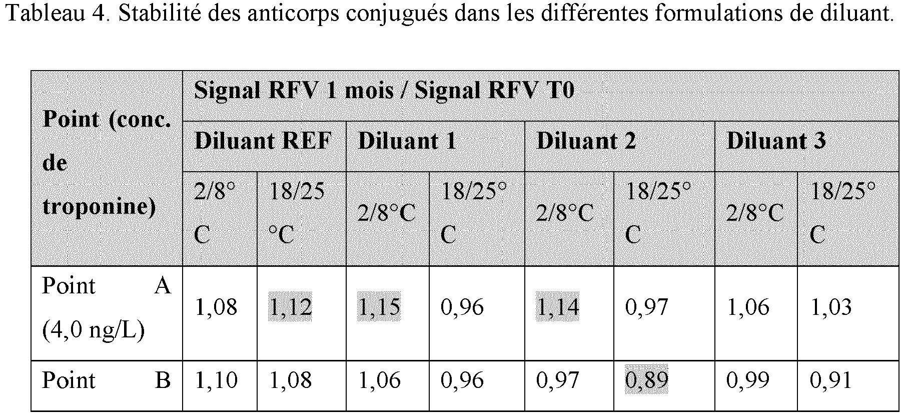

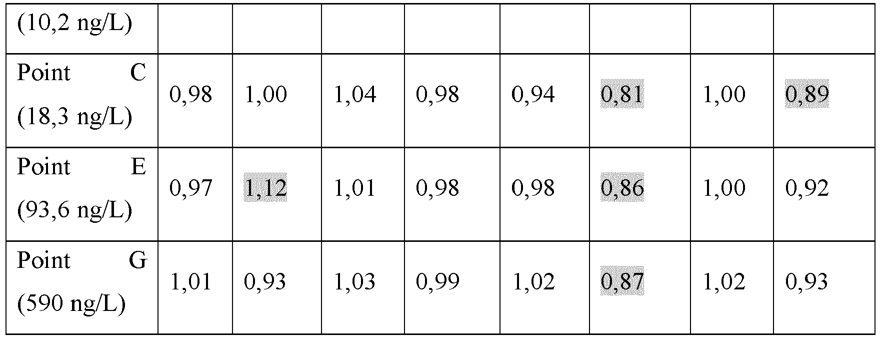

Les rapports >=1,10 ou= <0,90 sont surlignés en gris.

Pour qu'une formulation d'anticorps conjugué soit considérée comme stable, les rapports de signaux doivent être majoritairement inclus dans l'intervalle [0,90-1,10]. Le Diluant 2 qui contient le Biolipidure 206 ne permet pas d'obtenir des valeurs acceptables de stabilité en l'absence de sérum de cheval (agent stabilisant comparatif présent dans le Diluant REF). En revanche, l'ajout du PMAL C12 au Biolipidure 206 permet d'obtenir des valeurs satisfaisantes sur les différentes concentrations testées (voir Diluant 3).

### e) Effet du polymère PMAL C8

Le Diluant 4 a la même composition que le Diluant 3 (Tableau 2), sauf que le PMAL C12 du Diluant 3 est remplacé par du PMAL C8 dans le Diluant 4. Les deux diluants ont été comparés en utilisant un nouveau panel d'échantillons contenant des concentrations connues de Troponine I. Ces échantillons ont été testés par immunodosage sur VIDAS selon le mode opératoire décrit en c) avec la modification qui suit. Les anticorps conjugués ont été dilués chacun à une concentration de 0,9 µg/mL, soit dans le Diluant 3, soit dans le Diluant 4. Les résultats obtenus (signaux RFV) sont présentés dans le Tableau 5.

**Tableau 5. Comparaison des diluants de l'invention contenant du PMAL C12 ou du PMAL C8.**

| **Conc. Troponine I (ng/L)** | **Solution de conjugué avec Diluant 3** | **Solution de conjugué avec Diluant 4** |
|---|---|---|
| | **(PMAL C12 à 0,02%)** | **(PMAL C8 à 0,02%)** |
| 0,001 | 8 | 8 |
| 2,83 | 20 | 17 |
| 10,0 | 45 | 42 |
| 21,8 | 77 | 71 |
| 49,7 | 163 | 152 |

Le Diluant 3 contenant du PMAL C12 et le Diluant 4 contenant du PMAL C8 donnent des signaux RFV équivalents. Le PMAL C8 présente donc le même effet synergique inattendu que le PMAL C12.

### f) Effet synergique du PMAL C12 et du Biolipidure 206 pour les lots d'anticorps conjugués non conformes

Suivant les conditions de purification, les conditions de stockage ou toute autre raison, certains lots d'anticorps conjugués peuvent présenter un bruit de fond augmenté, de l'ordre de 15-20 RFV au lieu des 4-8 RFV observés habituellement. Le contrôle qualité effectué avant la libération des lots permet de rejeter de tels lots non conformes d'anticorps conjugués qui ne seront pas utilisés dans le produit final (la trousse d'immunoessai).

**Tableau 7. Impact des concentrations de PMAL C12 et Biolipidure 206 du Diluant 3 sur les ratios signal/bruit des lots d'anticorps conjugués non conformes.**

| **Item** | **Concentration de PMAL C12 + Biolipidure 206** | | |
|---|---|---|---|
| | **0,02% + 0,05% (Diluant 3)** | **0,02% + 0,1%** | **0,05% + 0,1%** |
| Signal RFV point O (blanc) | 16,0 | 12,0 | 8,9 |
| Signal RFV point A (4.0 ng/L) | 32 | 31 | 30 |
| RFV A / RFV O | 2,0 | 2,6 | 3,4 |
| RFV A - RFV O | 16,0 | 19,0 | 21,1 |
| Signal RFV point B (10.2 ng/L) | 43,7 | 31,0 | 30,0 |
| RFV B / RFV O | 2,7 | 3,8 | 4,9 |
| RFV B - RFV O | 27,7 | 33,5 | 34,6 |

En utilisant le Diluant 3 qui contient 0,02% de PMALC12 et 0,05% de Biolipidure 206, on observe un bruit de fond de 16 RFV avec deux lots d'anticorps conjugués non conformes. En augmentant la concentration de PMALC12 à 0,05% et celle de Biolipidure 206 à 0,1%, il est possible de réduire ce bruit de fond à 8,9 RFV, ce qui permet une amélioration considérable du ratio signal/bruit et rend envisageable une utilisation de tels lots dans un immunoessai. Le bruit de fond initial plus élevé de ces lots non-conformes nécessite des concentrations plus élevées de PMAL C12 et de Biolipidure 206 pour la mise en évidence de leur effet synergique bloquant.

## Revendications

1. Composition pour immunoessai, comprenant au moins (i) un amphipol choisi parmi
- les poly(anhydride maléiques) sur lesquels sont greffés au moins une chaine alkyle hydrophobe et au moins un groupe hydrophile, de masse moléculaire moyenne comprise entre 1 000 et 100 000 g/mol, de préférence entre 4 000 et 70 000 g/mol, ladite chaine alkyle hydrophobe étant une chaine alkyle linéaire de formule (-CH2)ₙ- avec n est un entier supérieur ou égal à 4, de préférence supérieur ou égal à 6, de préférence supérieur ou égal à 8, et par exemple compris entre 4 et 16, de préférence entre 8 et 12, et
(ii) un copolymère amphotérique à base de monomères de (méth)acrylate, une partie de ces monomères comprenant un groupe phosphorylcholine.

2. Composition selon la revendication 1, **caractérisée en ce que** le groupe hydrophile est un groupe carboxylate ou un groupe chargé positivement, en particulier un ammonium alkylé, par exemple la 3-(diméthylamino)-1-propylamine.

3. Composition selon l'une quelconque des revendications 1 à 2, **caractérisée en ce que** l'amphipol est choisi parmi les poly(anhydride-alt-1-octadécène maléiques), poly(anhydride-alt-1-tétradécène maléiques), poly(anhydride-alt-1-décène maléiques), substitués par un groupe hydrophile, notamment substitués par un ammonium alkylé, par exemple la 3-(diméthylamino)-1-propylamine.

4. Composition selon l'une quelconque des revendications 1 à 3, **caractérisée en ce que** l'amphipol est choisi parmi
- le poly(anhydride-*alt*-1-tétradécène maléique) substitué de formule (IIa) suivante : 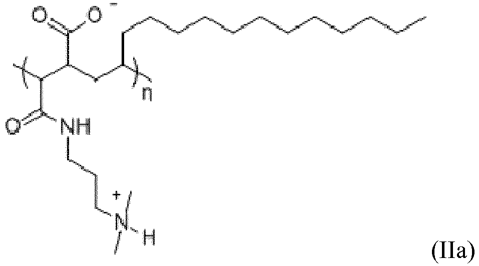 et de masse moléculaire moyenne comprise entre 10 000 et 14 000 g/mol, par exemple environ 12 000 g/mol,
- le poly(anhydride-*alt*-1-décène maléique) substitué de formule (IIb) suivante : 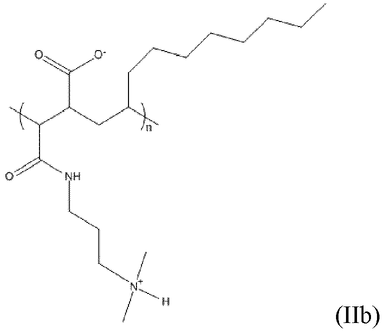 et de masse moléculaire moyenne comprise entre 16 000 et 20 000 g/mol, par exemple environ 18 500 g/mol, et
- le poly(anhydride-*alt*-1-octadécène maléique) substitué de formule (IIc) suivante : 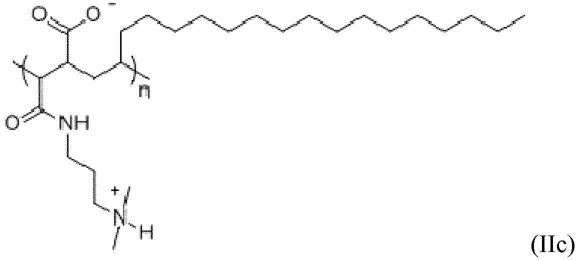 et de masse moléculaire moyenne comprise entre 39 000 et 65 000 g/mol.

5. Composition selon l'une des revendications 1 à 4, **caractérisée en ce que** le copolymère amphotérique est un copolymère amphotérique à base d'un monomère de 2-methacryloyloxyéthyl phosphorylcholine (MPC) et d'un autre monomère de méthacrylate fonctionnalisé avec un groupe hydrophobe, un groupe anionique, un groupe cationique ou un groupe permettant la formation de liaisons hydrogène.

6. Composition selon l'une des revendications 1 à 5, **caractérisée en ce que** le copolymère amphotérique est un copolymère amphotérique à base de MPC et de méthacrylate fonctionnalisé avec un groupe hydrophobe choisi parmi
- les alkyles linéaires ou ramifiés en C₄ à C₃₀ ;
- les alkyles linéaires ou ramifiés en C₄ à C₃₀ comprenant au moins un cycloalkyle ;
- les chaînes hydrocarbonées en C₄ à C₃₀ comprenant au moins une double liaison ;
- les chaînes hydrocarbonées en C₄ à C₃₀ comprenant au moins une triple liaison ; et,
- les chaînes hydrocarbonées en C₄ à C₃₀ comprenant au moins un cycle aromatique.

7. Composition selon l'une des revendications 1 à 6, **caractérisée en ce que** le copolymère amphotérique est le Biolipidure™ 206 commercialisé par la société NOF CORPORATION.

8. Solution aqueuse comprenant la composition selon l'une quelconque des revendications 1 à 7 et au moins un réactif d'immunoessai.

9. Solution aqueuse selon la revendication 8, **caractérisée en ce qu'**elle ne comprend pas d'agent stabilisant et/ou d'agent bloquant d'origine humaine ou animale, par exemple choisi parmi les sérums animaux, le lait écrémé, les caséines hydrolysées ou non, l'albumine bovine ou humaine, et les gélatines.

10. Trousse d'immunoessai comprenant au moins une solution aqueuse selon l'une des revendications 8 à 9, et au moins un partenaire de liaison à un analyte à détecter ou quantifier par immunoessai et, le cas échéant, les composés nécessaires à la mise en évidence d'une interaction spécifique entre le ou les partenaires de liaison et l'analyte.

11. Utilisation d'une composition telle que définie dans l'une des revendications 1 à 7 ou d'une solution aqueuse telle que définie dans l'une des revendications 8 à 9, dans un procédé par immunoessai, comme agent stabilisant et/ou bloquant.

12. Utilisation selon la revendication 11, pour réduire l'interaction non-spécifique entre (i) au moins un partenaire de liaison destiné à détecter ou quantifier un analyte dans un échantillon biologique susceptible de le contenir, par exemple un partenaire de liaison marqué, et, (ii) une phase solide.

13. Procédé par immunoessai de détermination de la présence ou de quantification d'un analyte cible, dans un échantillon biologique susceptible de le contenir, comprenant la mise en contact dudit échantillon avec au moins deux partenaires de liaison audit analyte, P1 et P2, séquentiellement ou simultanément, l'un des deux partenaires P2 étant marqué pour révéler la présence dudit analyte, s'il est présent, l'autre partenaire P1 étant de préférence immobilisé sur une phase solide, et ledit partenaire P2 marqué étant contenu dans au moins une composition telle que définie dans l'une des revendications 1 à 7 ou au moins une solution aqueuse telle que définie dans l'une des revendications 8 à 9, pour former le cas échéant un complexe P1/analyte/P2, la révélation de la présence dudit complexe formé permettant de conclure à la présence ou à la quantité d'analyte dans l'échantillon.

14. Procédé par immunoessai de détermination de la présence ou de quantification d'un analyte cible, dans un échantillon biologique susceptible de le contenir, comprenant la mise en contact dudit échantillon avec au moins un partenaire P1 de liaison audit analyte et un réactif R1 de compétition à l'analyte, ledit partenaire de liaison P1 étant de préférence immobilisé sur une phase solide, ledit réactif R1 étant marqué pour révéler indirectement la présence dudit analyte, et ledit réactif R1 étant contenu dans au moins une composition telle que définie dans l'une des revendications 1 à 7 ou au moins une solution aqueuse telle que définie dans l'une des revendications 8 à 9, pour former un complexe P1/R1, la révélation de la présence dudit complexe formé permettant de conclure indirectement à l'absence ou à la quantité d'analyte dans l'échantillon.

## Patentansprüche

1. Immunoassay-Zusammensetzung, umfassend mindestens (i) ein Amphipol, ausgewählt aus
- Poly(maleinsäureanhydrid), auf das mindestens eine hydrophobe Alkylkette und mindestens eine hydrophile Gruppe mit einem mittleren Molekulargewicht zwischen 1000 und 100.000 g/mol, vorzugsweise zwischen 4000 und 70.000 g/mol, aufgepfropft sind, wobei die hydrophobe Alkylkette eine lineare Alkylkette der Formel (-CH₂)ₙ- ist, in der n eine ganze Zahl größer oder gleich 4, vorzugsweise größer oder gleich 6, besonders bevorzugt größer oder gleich 8, und beispielsweise zwischen 4 und 16, vorzugsweise zwischen 8 und 12, ist, und
(ii) ein amphoteres Copolymer auf Basis von (Meth)acrylatmonomeren, wobei ein Teil der Monomere eine Phosphorylcholingruppe umfasst.

2. Zusammensetzung nach Anspruch 1, **dadurch gekennzeichnet, dass** die hydrophile Gruppe eine Carboxylatgruppe oder eine positiv geladene Gruppe ist, insbesondere ein alkyliertes Ammonium, zum Beispiel 3-(Dimethylamino)-1-propylamin.

3. Zusammensetzung nach einem der Ansprüche 1 bis 2, **dadurch gekennzeichnet, dass** das Amphipol ausgewählt ist aus hydrophil substituiertem Poly(maleinsäureanhydrid-alt-1-octadecen), Poly(maleinsäureanhydrid-alt-1-tetradecen), Poly(maleinsäureanhydrid-alt-1-decen), wobei das Amphipol durch eine hydrophile Gruppe, insbesondere ammoniumsubstituiertem Alkylgruppe substituiert ist, zum beispielsweise 3-(Dimethylamino)-1-propylamin.

4. Zusammensetzung nach einem der Ansprüche 1 bis 3, **dadurch gekennzeichnet, dass** das Amphipol ausgewählt ist aus
- substituiertes Poly(maleinsäureanhydrid-alt-1-tetradecen) der folgenden Formel (IIa) 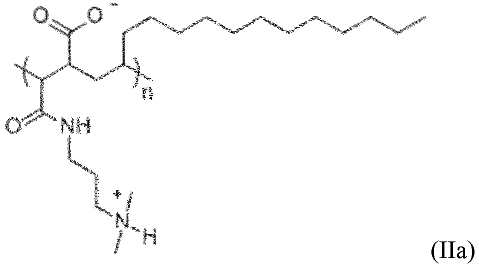 und mit einem mittleren Molekulargewicht zwischen 10.000 und 14.000 g/mol, zum Beispiel etwa 12.000 g/mol,
- substituiertes Poly(anhydrid-alt-1-decenylmaleat) mit der folgenden Formel (IIb) 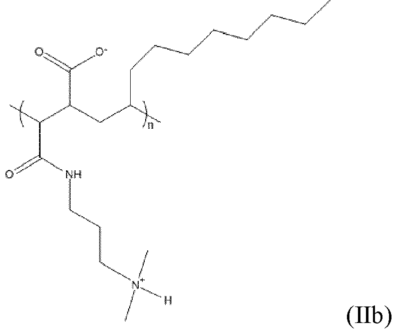 und mit einem mittleren Molekulargewicht zwischen 16.000 und 20.000 g/mol, zum Beispiel etwa 18.500 g/mol, und
- substituiertes Poly(maleinsäureanhydrid-1-octadecen) der folgenden Formel (IIc) 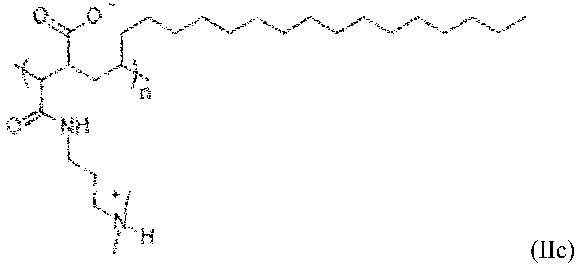 und mit einem durchschnittlichen Molekulargewicht zwischen 39.000 und 65.000 g/mol.

5. Zusammensetzung nach einem der Ansprüche 1 bis 4, **dadurch gekennzeichnet, dass** das amphotere Copolymer ein amphoteres Copolymer auf der Basis eines 2-Methacryloyloxyethylphosphorylcholin (MPC)-Monomers und eines anderen Methacrylatmonomers ist, das mit einer hydrophoben Gruppe, einer anionischen Gruppe, einer kationischen Gruppe oder einer Wasserstoffbrückenbindungsgruppe funktionalisiert ist.

6. Zusammensetzung nach einem der Ansprüche 1 bis 5, **dadurch gekennzeichnet, dass** das amphotere Copolymer ein amphoteres Copolymer auf Basis von MPC und Methacrylat ist, das mit einer hydrophoben Gruppe funktionalisiert ist, die ausgewählt ist aus
- lineare oder verzweigte C₄- bis C₃₀-Alkyle;
- lineare oder verzweigte C₄- bis C₃₀-Alkyle mit mindestens einem Cycloalkyl ;
- C₄- bis C₃₀-Kohlenwasserstoffketten, die mindestens eine Doppelbindung enthalten;
- wobei die C₄- bis C₃₀-Kohlenwasserstoffketten mindestens eine Dreifachbindung umfassen; und,
- wobei die C₄- bis C₃₀-Kohlenwasserstoffketten mindestens einen aromatischen Ring umfassen.

7. Zusammensetzung nach einem der Ansprüche 1 bis 6, **dadurch gekennzeichnet, dass** das amphotere Copolymer Biolipidure™ 206 ist, das von der Firma NOF CORPORATION vertrieben wird.

8. Wässrige Lösung, umfassend die Zusammensetzung nach einem der Ansprüche 1 bis 7 und mindestens ein Immunoassay-Reagenz.

9. Wässrige Lösung nach Anspruch 8, **dadurch gekennzeichnet, dass** sie kein Stabilisierungsmittel und/oder Blockierungsmittel menschlichen oder tierischen Ursprungs enthält, beispielsweise ausgewählt aus tierischen Seren, Magermilch, hydrolysierten oder nicht-hydrolysierten Kaseinen, Rinder- oder Humanalbumin und Gelatinen.

10. Immunoassay-Kit, umfassend mindestens eine wässrige Lösung nach einem der Ansprüche 8 bis 9 und mindestens einen Bindungspartner zu einem mittels Immunoassay nachzuweisenden oder zu quantifizierenden Analyten und gegebenenfalls die zum Nachweis einer spezifischen Wechselwirkung zwischen dem/den Bindungspartner(n) und dem Analyten erforderlichen Verbindungen.

11. Verwendung einer Zusammensetzung nach einem der Ansprüche 1 bis 7 oder einer wässrigen Lösung nach einem der Ansprüche 8 bis 9 in einem Immunoassay-Verfahren als Stabilisierungs- und/oder Blockierungsmittel.

12. Verwendung nach Anspruch 11 zur Verringerung der unspezifischen Wechselwirkung zwischen (i) mindestens einem Bindungspartner zum Nachweis oder zur Quantifizierung eines Analyten in einer biologischen Probe, die diesen wahrscheinlich enthält, z.B. einem markierten Bindungspartner, und (ii) einer festen Phase.

13. Verfahren zur Bestimmung des Vorhandenseins oder der Quantifizierung eines Zielanalyten in einer biologischen Probe, die ihn enthalten kann, durch Immunoassay, umfassend das Inkontaktbringen der Probe mit mindestens zwei Partnern für die Bindung an den Analyten, P1 und P2, nacheinander oder gleichzeitig, wobei einer der beiden Partner P2 markiert ist, um das Vorhandensein des Analyten, falls er vorhanden ist, zu zeigen, und der andere Partner P1 vorzugsweise auf einer festen Phase immobilisiert ist, und wobei der markierte Partner P2 in mindestens einer Zusammensetzung gemäß einem der Ansprüche 1 bis 7 oder mindestens einer wässrigen Lösung gemäß einem der Ansprüche 8 bis 9 enthalten ist, um gegebenenfalls einen P1/Analyt/P2-Komplex zu bilden, wobei die Offenbarung des Vorhandenseins des gebildeten Komplexes einen Rückschluss auf das Vorhandensein oder die Menge des Analyten in der Probe ermöglicht.

14. Verfahren zur Bestimmung des Vorhandenseins oder der Quantifizierung eines Zielanalyten in einer biologischen Probe, die ihn enthalten kann, durch Immunoassay, umfassend das Inkontaktbringen der Probe mit mindestens einem Bindungspartner P1 für den Analyten und einem Reagens R1, das mit dem Analyten konkurriert, wobei der Bindungspartner P1 vorzugsweise auf einer festen Phase immobilisiert ist und das Reagens R1 markiert ist, um indirekt das Vorhandensein des Analyten zu zeigen, und das Reagenz R1 in mindestens einer Zusammensetzung gemäß einem der Ansprüche 1 bis 7 oder mindestens einer wässrigen Lösung gemäß einem der Ansprüche 8 bis 9 enthalten ist, um einen P1/R1-Komplex zu bilden, wobei der Nachweis des Vorhandenseins des gebildeten Komplexes indirekt auf das Fehlen oder die Menge des Analyten in der Probe schließen lässt.

## Claims

1. Composition for immunoassay, comprising at least (i) an amphipol selected from
- the poly(maleic anhydride)s, on which at least one hydrophobic alkyl chain and at least one hydrophilic group are grafted, with an average molecular weight comprised between 1,000 and 100,000 g/mol, preferably between 4,000 and 70,000 g/mol, said hydrophobic alkyl chain being a linear alkyl chain of formula (-CH₂)ₙ- where n is an integer greater than or equal to 4, preferably greater than or equal to 6, preferably greater than or equal to 8, and for example comprised between 4 and 16, preferably between 8 and 12,and
(ii) an amphoteric copolymer based on (meth)acrylate monomers, a part of these monomers comprising a phosphorylcholine group.

2. Composition according to claim 1, **characterized in that** the hydrophilic group is a carboxylate group or a positively charged group, in particular an alkylated ammonium, for example 3-(dimethylamino)-1-propylamine.

3. Composition according to any one of claims 1 to 2, **characterized in that** the amphipol is selected from poly(maleic anhydride-*alt*-1-octadecene)s, poly(maleic anhydride-*alt*-1-tetradecene)s, poly(maleic anhydride-*alt*-1-decene)s, substituted with a hydrophilic group, in particular substituted with an alkylated ammonium, for example 3-(dimethylamino)-1-propylamine.

4. Composition according to any one of claims 1 to 3, **characterized in that** the amphipol is selected from
- the substituted poly(maleic anhydride-*alt*-1-tetradecene) of the following formula (IIa): 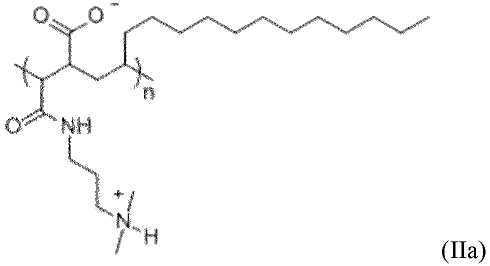 with an average molecular weight comprised between 10,000 and 14,000 g/mol, for example approximately 12,000 g/mol,
- the substituted poly(maleic anhydride-*alt*-1-decene) of the following formula (IIb) : 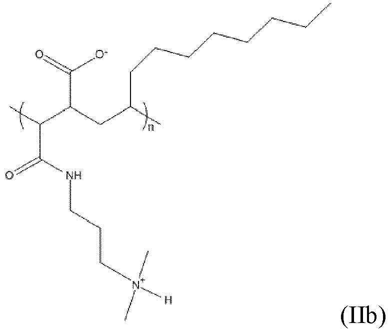 with an average molecular weight comprised between 16,000 and 20,000 g/mol, for example approximately 18,500 g/mol, and
- the substituted poly(maleic anhydride-*alt*-1-octadecene) of the following formula (IIc): 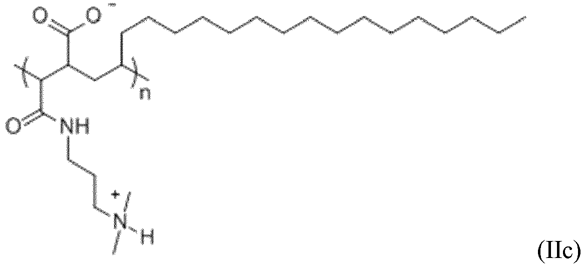 with an average molecular weight comprised between 39,000 and 65,000 g/mol.

5. Composition according to one of claims 1 to 4, **characterized in that** the amphoteric copolymer is an amphoteric copolymer based on a monomer of 2-methacryloyloxyethyl phosphorylcholine (MPC) and another methacrylate monomer functionalized with a hydrophobic group, an anionic group, a cationic group or a group allowing formation of hydrogen bonds.

6. Composition according to one of claims 1 to 5, **characterized in that** the amphoteric copolymer is an amphoteric copolymer based on MPC and methacrylate functionalized with a hydrophobic group selected from
- linear or branched C₄ to C₃₀ alkyls;
- linear or branched C₄ to C₃₀ alkyls comprising at least one cycloalkyl;
- C₄ to C₃₀ hydrocarbon-containing chains comprising at least one double bond;
- C₄ to C₃₀ hydrocarbon-containing chains comprising at least one triple bond; and,
- C₄ to C₃₀ hydrocarbon-containing chains comprising at least one aromatic ring.

7. Composition according to one of claims 1 to 6, **characterized in that** the amphoteric copolymer is Biolipidure™ 206 marketed by the company NOF CORPORATION.

8. Aqueous solution comprising the composition according to any one of claims 1 to 7 and at least one immunoassay reagent.

9. Aqueous solution according to claim 8, **characterized in that** it does not comprise a stabilizer and/or blocking agent of human or animal origin, for example selected from animal sera, skimmed milk, hydrolyzed or non-hydrolyzed caseins, bovine or human albumin, and gelatins.

10. Immunoassay kit comprising at least one aqueous solution according to one of claims 8 to 9, and at least one binding partner to an analyte to be detected or quantified by immunoassay and, if applicable, the compounds necessary for demonstrating a specific interaction between the binding partner or partners and the analyte.

11. Use of a composition as defined in one of claims 1 to 7 or of an aqueous solution as defined in one of claims 8 to 9, in an immunoassay method, as a stabilizer and/or blocking agent.

12. Use according to claim 11, for reducing the non-specific interaction between (i) at least one binding partner intended to detect or quantify an analyte in a biological sample that may contain it, for example a labelled binding partner, and (ii) a solid phase.

13. Immunoassay method for determining the presence or for quantification of a target analyte, in a biological sample that may contain it, comprising contacting said sample with at least two binding partners to said analyte, P1 and P2, sequentially or simultaneously, one of the two partners P2 being labelled to show the presence of said analyte, if it is present, the other partner P1 preferably being immobilized on a solid phase, and said labelled partner P2 being contained in at least one composition as defined in one of claims 1 to 7 or at least one aqueous solution as defined in one of claims 8 to 9, to form if applicable a P1/analyte/P2 complex, detection of the presence of said complex formed allowing conclusions to be drawn concerning the presence or the quantity of analyte in the sample.

14. Immunoassay method for determining the presence or for quantification of a target analyte, in a biological sample that may contain it, comprising contacting said sample with at least one binding partner P1 to said analyte and a reagent R1 that competes with the analyte, said binding partner P1 preferably being immobilized on a solid phase, said reagent R1 being labelled to show indirectly the presence of said analyte, and said reagent R1 being contained in at least one composition as defined in one of claims 1 to 7 or at least one aqueous solution as defined in one of claims 8 to 9, to form a P1/R1 complex, detection of the presence of said complex formed allowing conclusions to be drawn indirectly concerning the absence or the quantity of analyte in the sample.
